(19) Europäisches Patentamt
European Patent Office
Office européen des brevets

(11) EP 3 220 886 B1

(12) **EUROPEAN PATENT SPECIFICATION**

(45) Date of publication and mention of the grant of the patent:
**28.04.2021 Bulletin 2021/17**

(21) Application number: **15797958.4**

(22) Date of filing: **16.11.2015**

(51) Int Cl.:
***A61K 8/19*** *(2006.01)* ***A61K 8/34*** *(2006.01)*
***A61Q 5/10*** *(2006.01)* ***A61K 8/97*** *(2017.01)*
***A61K 8/46*** *(2006.01)* ***A61K 8/9789*** *(2017.01)*
***A61K 8/9794*** *(2017.01)* ***A61K 8/9767*** *(2017.01)*

(86) International application number:
**PCT/EP2015/076693**

(87) International publication number:
**WO 2016/079055 (26.05.2016 Gazette 2016/21)**

(54) **COMPOSITION COMPRISING AN ORTHO-DIPHENOL DERIVATIVE, AN ALKALI METAL (BI)CARBONATE, A WATER-SOLUBLE ORGANIC SOLVENT AND A REDUCING AGENT**

ZUSAMMENSETZUNG MIT EINEM ORTHODIPHENOLDERIVAT, EINEM ALKALIMETALL-(BI)CARBONAT, EINEM WASSERLÖSLICHEN ORGANISCHEN LÖSUNGSMITTEL UND EINEM REDUKTIONSMITTEL

COMPOSITION COMPRENANT UN DÉRIVÉ D'ORTHO-DIPHÉNOL, UN (BI)CARBONATE DE MÉTAL ALCALIN, UN SOLVANT ORGANIQUE SOLUBLE DANS L'EAU, ET UN AGENT DE RÉDUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2014 FR 1461058**

(43) Date of publication of application:
**27.09.2017 Bulletin 2017/39**

(73) Proprietor: **L'Oréal**
**75008 Paris (FR)**

(72) Inventor: **WAHLER, Arno**
**F-78600 Maisons Laffite (FR)**

(74) Representative: **L'Oreal**
**Service D.I.P.I.**
**9, rue Pierre Dreyfus**
**92110 Clichy (FR)**

(56) References cited:
**EP-A2- 2 196 188**
**WO-A1-2014/002670**
**CN-A- 102 824 283**
**FR-A1- 2 981 569**
**KR-A- 20110 060 379**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 220 886 B1

## Description

The present invention relates to a composition comprising at least one ortho-diphenol being chosen from natural ortho-diphenol chosen from extracts of tree wood, at least one alkali metal or alkaline-earth metal (bi)carbonate, at least one water-soluble organic solvent chosen from non-aromatic C2-C6 alcohol and at least one reducing agent chosen from alkali metal or alkaline-earth metal sulfites or bisulfites.

It is known practice to obtain "permanent" colourings with dye compositions comprising oxidation dye precursors, generally known as oxidation bases, such as ortho- or para-phenylenediamines, ortho- or para-aminophenols and heterocyclic compounds. These oxidation bases are colourless or weakly coloured compounds, which, when combined with oxidizing products, may give rise to coloured compounds via a process of oxidative condensation. It is also known that the shades obtained may be varied by combining these oxidation bases with couplers or colouring modifiers, the latter being chosen especially from aromatic meta-diamines, meta-aminophenols, meta-diphenols and certain heterocyclic compounds such as indole compounds. This oxidation dyeing process consists in applying to the keratin fibres bases or a mixture of bases and couplers with hydrogen peroxide ($H_2O_2$ or aqueous hydrogen peroxide solution), as oxidizing agent, in leaving it to diffuse, and then in rinsing the fibres. The colourings resulting therefrom are permanent, strong and resistant to external agents, especially to light, bad weather, washing, perspiration and rubbing.

However, commercial hair dyes that contain them may have drawbacks such as staining, and problems of odour, discomfort and degradation of the keratin fibres. This is particularly the case with oxidation dyeing operations.

In the field of dyeing, it is also known practice to dye keratin materials such as the hair or the skin using natural extracts such as ortho-diphenol compounds or extracts rich in these compounds.

Compositions comprising ortho-diphenol compounds in the presence of a metal salt, especially of Mn and/or of Zn, are found. In particular, patent applications FR 2 814 943, FR 2 814 945, FR 2 814 946 and FR 2 814 947 propose compositions for dyeing the skin or keratin fibres, comprising a dye precursor that contains at least one ortho-diphenol, Mn and/or Zn oxides and salts, alkaline agents of hydrogen carbonate type in a particular Mn, Zn/hydrogen carbonate ratio and optionally an enzyme. Patent applications FR 2 939 654, FR 2 939 644, FR 2 939 645 and FR 2 939 646 describe the use of metal salts, combined with ortho-diphenols, alkaline agents and a chemical oxidizing agent for dyeing keratin fibres.

However, the natural extracts are highly unstable in solution, which impairs their dyeing properties.

Patent application CN102824283 describes environmentally-friendly hair care colouring compositions composed of three agents **A**, **B** and **C**, **A** comprising L-cysteine, glycerin and sodium carbonate.

Patent application WO 201402670 describes compositions for dyeing keratin fibres, comprising a first agent comprising at least one compound chosen from tannic acid, gallic acid or derivatives thereof and an ammonium hydrogen carbonate and a second agent comprising a ferric salt. However, these compositions do not have entirely satisfactory properties, especially in terms of stability.

There is a need to develop improved compositions that have good stability over time while at the same time conserving good dyeing properties especially in terms of power and chromaticity.

This aim is achieved with the present invention, the subject of which is a composition comprising:

- at least one ortho-diphenol comprising at least one aromatic ring and comprising at least two hydroxyl groups (OH) borne by two adjacent carbon atoms of the aromatic ring, the content of ortho-diphenol being between 5% and 10% by weight relative to the total weight of the composition said ortho-diphenol being chosen from natural ortho-diphenol chosen from extracts of tree wood,
- at least one alkali metal or alkaline-earth metal (bi)carbonate compound, the alkali metal or alkaline-earth metal (bi)carbonate compound(s) representing from 0.001% to 20% by weight relative to the total weight of the composition
- at least one water-soluble organic solvent, the water-soluble organic solvent(s) representing from 10% to 50% by weight relative to the total weight of the composition, said solvent being chosen from non-aromatic $C_2$-$C_6$ alcohol and
- at least one reducing agent the reducing agent representing from 0.01% to 50% by weight relative to the total weight of the composition, the reducing agent being chosen from alkali metal or alkaline-earth metal sulfites or bisulfites.

The composition according to the invention shows good stability over time.

A subject of the invention is also a process for dyeing keratin fibres, in particular human keratin fibres such as the hair, comprising the application to the keratin fibres of the composition according to the invention.

A subject of the invention is also a process for dyeing keratin fibres, in particular human keratin fibres such as the hair, comprising the application to the keratin fibres of a first dye composition as disclosed above, and then of a second composition comprising at least one metal salt.

In the text hereinbelow, and unless otherwise indicated, the limits of a range of values are included within that range.

The expression "at least one" is equivalent to the expression "one or more".

**Ortho-diphenol derivative(s):**

The composition according to the invention comprises at least one ortho-diphenol chosen from extracts of the wood.

The composition according to the invention can comprise at least one ortho-diphenol derivative in a content between 5% to 10% by weight relative to the weight of the composition.

According to the invention, the term "ortho-diphenol derivatives" means compounds comprising at least one aromatic ring, preferably a benzene ring, and comprising at least two hydroxyl groups (OH) borne by two adjacent carbon atoms of the aromatic ring.

The term "mixtures" means a combination of several identical or different ortho-diphenol derivatives.

The aromatic ring is more particularly a fused aryl or fused heteroaryl ring, i.e. optionally containing one or more heteroatoms, such as benzene, naphthalene, tetrahydronaphthalene, indane, indene, anthracene, phenanthrene, isoindole, indoline, isoindoline, benzofuran, dihydrobenzofuran, benzopyran, dihydrobenzopyran, chroman, isochroman, chromene, isochromene, quinoline, tetrahydroquinoline and isoquinoline, said aromatic ring comprising at least two hydroxyl groups borne by two adjacent carbon atoms of the aromatic ring. Preferentially, the aromatic ring of the ortho-diphenol derivatives according to the invention is an aryl ring such as a benzene ring.

The term "fused ring" means that at least two saturated or unsaturated and heterocyclic or non-heterocyclic rings have a common bond, i.e. that at least one ring is joined side by side with another ring.

The ortho-diphenol derivatives according to the invention may or may not be salified. They may also be in the aglycone form (without bonded sugar) or in the form of glycosylated compounds (with bonded sugar).

More particularly, the ortho-diphenol derivative represents a compound of formula (I), or an oligomer thereof, in salified or non-salified form:

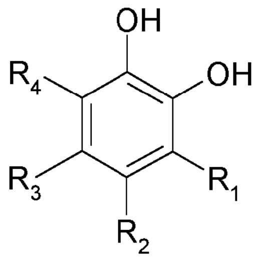

**(I)**

in which formula **(I):**

- **$R_1$** to **$R_4$,** which may be identical or different, represent an atom or a radical chosen from:
a) hydrogen; b) halogen; c) hydroxyl; d) carboxyl (C(O)OH) in acid or salified form (preferably with an alkali metal or a substituted or unsubstituted ammonium); e) alkyl carboxylate or alkoxycarbonyl; f) amino, which is optionally substituted, especially with one or two alkyl groups; g) optionally substituted linear or branched alkyl; h) optionally substituted linear or branched alkenyl; i) optionally substituted cycloalkyl; j) alkoxy; k) alkoxyalkyl; l) alkoxyaryl; m) optionally substituted aryl; n) saturated or unsaturated heterocycle, optionally bearing a cationic or anionic charge, optionally substituted and/or optionally fused with an aromatic, preferably benzene, ring, said aromatic ring being optionally substituted particularly with one or more hydroxyl or glycosyloxy groups; o) radical containing one or more silicon atoms; p) a glycosyloxy group;
- or else two of the substituents borne by two adjacent carbon atoms **$R_1$** - **$R_2$, $R_2$ - $R_3$ or $R_3$** - **$R_4$** form, together with the carbon atoms bearing them, a saturated or unsaturated and aromatic or non-aromatic ring optionally containing one or more heteroatoms and optionally fused with one or more saturated or unsaturated rings optionally containing one or more heteroatoms. Particularly, $R_1$ to $R_4$ together form from one to four rings.

According to a particular embodiment of the invention, the ortho-diphenol derivative(s) are of formula **(I)** and are such that **$R_3$** represents a heterocycle and **$R_1$, $R_2$** and **$R_4$** preferably represent a hydrogen atom, a hydroxyl group or a glycosyloxy group. Preferentially, the heterocyclic group is a 10-membered bicyclic group, comprising at least one oxygen atom and at least one benzo group, said heterocycle being optionally unsaturated, and/or optionally substituted with at least one group chosen from hydroxyl, glycosyloxy, an oxo group and arylcarbonyloxy such as 3,4,5-trihydroxyphenyl-1-carbonyloxy (-O-gallate), the aryl group being optionally substituted with at least one hydroxyl group.

The saturated or unsaturated and optionally fused rings can also be optionally substituted.

The "*alkyl*" radicals are saturated, linear or branched, generally $C_1$-$C_{20}$ and particularly $C_1$-$C_{10}$ hydrocarbon-based radicals, preferably $C_1$-$C_6$ alkyl radicals, such as methyl, ethyl, propyl, butyl, pentyl and hexyl.

The "*alkenyl*" radicals are unsaturated, linear or branched, $C_2$-$C_{20}$ hydrocarbon-based radicals; preferably comprising at least one double bond, such as ethylene, propylene, butylene, pentylene, 2-methylpropylene and decylene.

The "*aryl*" radicals are fused or non-fused monocyclic or polycyclic carbon-based radicals, preferentially comprising from 6 to 30 carbon atoms, and of which at least one ring is aromatic; the aryl radical according to the invention is particularly chosen from a phenyl, biphenyl, naphthyl, indenyl, anthracenyl or tetrahydronaphthyl; more preferentially, the aryl radical according to the invention is a phenyl.

The "*alkoxy*" radicals are alkyl-oxy radicals with alkyl as defined previously, preferably $C_1$-$C_{10}$ alkyl, such as methoxy, ethoxy, propoxy and butoxy.

The "*alkoxyalkyl*" radicals are preferably ($C_1$-$C_{20}$)alkoxy($C_1$-$C_{20}$)alkyl radicals, such as methoxymethyl, ethoxymethyl, methoxyethyl, ethoxyethyl, and the like.

The "*cycloalkyl*" radicals are generally $C_4$-$C_8$ cycloalkyl radicals, preferably cyclopentyl and cyclohexyl radicals. The cycloalkyl radicals can be substituted cycloalkyl radicals, in particular substituted by alkyl, alkoxy, carboxylic acid, hydroxyl, amine and ketone groups.

The "*alkyl*" or "*alkenyl*" radicals, when they are "*optionally substituted*", may be substituted with at least one substituent borne by at least one carbon atom chosen from: a) halogen; b) hydroxyl; c) $C_1$-$C_2$ alkoxy; d) $C_1$-$C_{10}$ alkoxycarbonyl; e) (poly)hydroxy($C_2$-$C_4$)alkoxy; f) amino; g) 5- or 6-membered heterocycloalkyl; h) optionally cationic 5- or 6-membered heteroaryl, preferably imidazolium, optionally substituted with a ($C_1$-$C_4$)alkyl radical, preferentially methyl; i) amino substituted with one or two identical or different $C_1$-$C_6$ alkyl radicals optionally bearing at least: 1) one hydroxyl group, 2) one amino group optionally substituted with one or two optionally substituted $C_1$-$C_3$ alkyl radicals, it being possible for said alkyl radicals to form, with the nitrogen atom to which they are attached, a saturated or unsaturated and optionally substituted 5- to 7-membered heterocycle optionally comprising at least one other nitrogen or non-nitrogen heteroatom, 3) one quaternary ammonium group $-N^+R'R''R'''$, M- for which R', R" and R'", which may be identical or different, represent a hydrogen atom or a $C_1$-$C_4$ alkyl group; and $M^-$ represents the counterion of the corresponding organic acid, mineral acid or halide, 4) or one optionally cationic 5- or 6-membered heteroaryl radical, preferentially imidazolium, optionally substituted with a ($C_1$-$C_4$)alkyl radical, preferably methyl; j) acylamino (-N(R)-C(O)-R') in which the radical R is a hydrogen atom or a ($C_1$-$C_4$)alkyl radical optionally bearing at least one hydroxyl group and the radical R' is a $C_1$-$C_2$ alkyl radical; k) carbamoyl ($(R)_2N$-C(O)-) in which the radicals R, which may be identical or different, represent a hydrogen atom or a $C_1$-$C_4$ alkyl radical optionally bearing at least one hydroxyl group; l) alkylsulfonylamino (R'-$S(O)_2$-N(R)-) in which the radical R represents a hydrogen atom or a $C_1$-$C_4$ alkyl radical optionally bearing at least one hydroxyl group and the R' radical represents a $C_1$-$C_4$ alkyl radical, a phenyl radical; m) aminosulfonyl ($(R)_2N$-$S(O)_2$-) in which the radicals R, which may be identical or different, represent a hydrogen atom or a $C_1$-$C_4$ alkyl radical optionally bearing at least one hydroxyl group; n) carboxyl in acid or salified form (preferably salified with an alkali metal or a substituted or unsubstituted ammonium); o) cyano; p) nitro; q) carboxyl or glycosylcarbonyl; r) phenylcarbonyloxy optionally substituted with one or more hydroxyl groups; s) glycosyloxy; and t) phenyl optionally substituted with one or more hydroxyl groups.

The "aryl" or "heterocyclic" radicals or the "aryl" or "heterocyclic" part of the radicals, when they are "optionally substituted" may be substituted with at least one substituent borne by at least one carbon atom chosen from a) $C_1$-$C_{10}$ alkyl, preferably $C_1$-$C_8$ alkyl, optionally substituted with one or more radicals chosen from the following radicals: hydroxyl, $C_1$-$C_2$ alkoxy, (poly)hydroxy($C_2$-$C_4$)alkoxy, acylamino, amino substituted with two identical or different $C_1$-$C_4$ alkyl radicals optionally bearing at least one hydroxyl group or it being possible for the two radicals to form, with the nitrogen atom to which they are attached, a saturated or unsaturated and optionally substituted 5- to 7-membered, preferably 5- or 6-membered, heterocycle optionally comprising another nitrogen or non-nitrogen heteroatom; b) halogen; c) hydroxyl; d) $C_1$-$C_2$ alkoxy; e) $C_1$-$C_{10}$ alkoxycarbonyl; f) (poly)hydroxy($C_2$-$C_4$)alkoxy; h) amino; i) 5- or 6-membered heterocycloalkyl; j) optionally cationic 5- or 6-membered heteroaryl, preferably imidazolium, optionally substituted with a ($C_1$-$C_4$)alkyl radical, preferentially methyl; k) amino substituted with one or two identical or different $C_1$-$C_6$ alkyl radicals optionally bearing at least 1) hydroxyl, 2) amino optionally substituted with one or two optionally substituted $C_1$-$C_3$ alkyl radicals, it being possible for said alkyl radicals to form, with the nitrogen atom to which they are attached, a saturated or unsaturated and optionally substituted 5- to 7-membered heterocycle optionally comprising at least one other nitrogen or non-nitrogen heteroatom, 3) quaternary ammonium $-N^+R'R''R'''$, $M^-$ for which R', R" and R'", which may be identical or different, represent a hydrogen atom or a $C_1$-$C_4$ alkyl group; and $M^-$ represents the counterion of the corresponding organic acid, mineral acid or halide, 4) or optionally cationic 5- or 6-membered heteroaryl, preferentially imidazolium, optionally substituted with a ($C_1$-$C_4$)alkyl radical, preferentially methyl; l) an acylamino radical (-N(R)-C(O)-R') in which the radical R is a hydrogen atom or a $C_1$-$C_4$ alkyl radical optionally bearing at least one hydroxyl group and the radical R' is a $C_1$-$C_2$ alkyl radical; a carbamoyl radical ($(R)_2N$-C(O)-) in which the radicals R, which may be identical or different, represent a hydrogen atom or a $C_1$-$C_4$ alkyl radical optionally bearing at least one hydroxyl group; an alkylsulfonylamino radical (R'$S(O)_2$-N(R)-) in which the radical R represents a hydrogen atom or a $C_1$-$C_4$ alkyl radical optionally bearing at least

one hydroxyl group and the radical R' represents a $C_1$-$C_4$ alkyl radical, a phenyl radical; an aminosulfonyl radical ($(R)_2N$-$S(O)_2$-) in which the radicals R, which may be identical or different, represent a hydrogen atom or a $C_1$-$C_4$ alkyl radical optionally bearing at least one hydroxyl group; m) carboxyl (C(O)OH) in acid or salified form (preferably salified with an alkali metal or a substituted or unsubstituted ammonium); n) a cyano group; o) a nitro group ($N(O)_2$); p) a polyhaloalkyl group, preferentially trifluoromethyl; q) a carboxyl or glycosylcarbonyl group; r) a phenylcarbonyloxy group optionally substituted with one or more hydroxyl groups; s) a glycosyloxy group; and t) a phenyl group optionally substituted with one or more hydroxyl groups.

The term "*glycosyl* radical" means a radical derived from a monosaccharide or polysaccharide.

The radicals comprising one or more silicon atoms are preferably polydimethylsiloxane, polydiphenylsiloxane, polydimethylphenylsiloxane or stearoxy dimethicone radicals.

The "*heterocyclic*" radicals are generally radicals comprising, in at least one ring, one or more heteroatoms chosen from O, N and S, preferably O or N, optionally substituted in particular with one or more alkyl, alkoxy, carboxylic acid, hydroxyl, amine or ketone groups. These rings may contain one or more oxo groups on the carbon atoms of the heterocycle.

Mention may be made, among the heterocyclic radicals that may be used, of the furyl, pyranyl, pyrrolyl, imidazolyl, pyrazolyl, pyridyl or thienyl groups.

More preferably, the heterocyclic groups are fused groups such as benzofuryl, benzopyranyl, dihydrobenzopyranyl, chromenyl, xanthenyl, indolyl, isoindolyl, quinolyl, isoquinolyl, chromanyl, isochromanyl, indolinyl, isoindolinyl, coumarinyl or isocoumarinyl groups, these groups possibly being substituted, in particular with one or more OH groups.

The ortho-diphenols that are useful in the composition according to the invention may be natural or synthetic. Among the natural ortho-diphenols are compounds that may be present in nature and that are reproduced by chemical (semi)synthesis. When they are natural, they are usually a mixture of ortho-diphenols.

The salts of the ortho-diphenols of the invention may be salts of acids or of bases. The acids may be mineral or organic. Preferably, the acid is hydrochloric acid, which results in chlorides.

The bases may be mineral or organic. In particular, the bases are alkali metal hydroxides, such as sodium hydroxide, which results in sodium salts.

According to a particular embodiment of the invention, the dye composition comprises as compound one or more synthetic ortho-diphenol derivative(s) that do not exist in nature.

According to another preferred embodiment of the invention, the composition comprises as compound one or more natural ortho-diphenol derivative(s).

More particularly, the ortho-diphenols that may be used in the composition according to the invention are in particular chosen from:

- flavanols such as catechin and epicatechin gallate;
- flavonols such as quercetin;
- anthocyanidins, such as cyanidin, delphinidin and petunidin;
- anthocyanins or anthocyans, for instance myrtillin;
- ortho-hydroxybenzoates, for example gallic acid salts;
- flavones, such as luteolin;
- hydroxystilbenes, for example 3,3',4,5'-tetrahydroxystilbene, optionally oxylated (for example glucosylated);
- 3,4-dihydroxyphenylalanine and derivatives thereof;
- 2,3-dihydroxyphenylalanine and derivatives thereof;
- 4,5-dihydroxyphenylalanine and derivatives thereof;
- dihydroxycinnamates, such as caffeic acid and chlorogenic acid;
- ortho-polyhydroxycoumarins;
- ortho-polyhydroxyisocoumarins;
- ortho-polyhydroxycoumarones;
- ortho-polyhydroxyisocoumarones;
- ortho-polyhydroxychalcones;
- ortho-polyhydroxychromones;
- quinones;
- hydroxyxanthones;
- 1,2 dihydroxybenzene and derivatives thereof;
- 1,2,4-trihydroxybenzene and derivatives thereof;
- 1,2,3-trihydroxybenzene and derivatives thereof;
- 2,4,5-trihydroxytoluene and derivatives thereof;
- proanthocyanidins and especially the proanthocyanidins A1, A2, B1, B2, B3 and C1;
- proanthocyanins;

- tannic acid;
- gallic acid;
- ellagic acid;
- neoflavanols and neoflavanones such as derivatives of haematoxylin, of haematin, of brazilin and of brazilein; and mixtures of the preceding compounds.

According to one embodiment of the invention, the ortho-diphenols of the invention are chosen from flavanols such as catechins and epicatechin gallate, flavonols such as quercetin, ortho-hydroxybenzoates, for example gallic acid salts, proanthocyanidins and especially the proanthocyanidins A1, A2, B1, B2, B3 and C1, tannic acid, gallic acid, neoflavanols and neoflavanones such as derivatives of haematoxylin, of haematin, of brazilin and of brazilein, and mixtures of the preceding compounds.

More particularly, the ortho-diphenol derivatives may be chosen from the dihydroxyflavonoids of formula **(II)** and also organic or mineral acid or base salts thereof, optical, geometric and tautomeric isomers thereof, and solvates thereof such as hydrates:

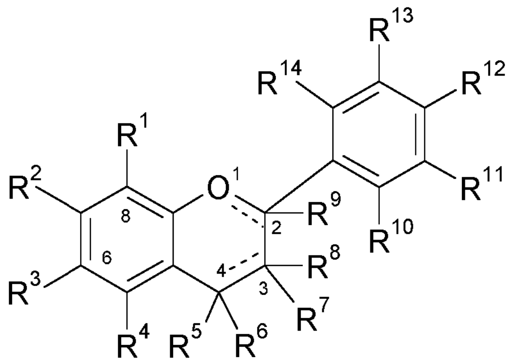

**(II)**

in which formula **(II):**

- ---- represents a single bond or a double bond;
- **$R^1$, $R^2$, $R^3$, $R^4$, $R^{10}$, $R^{11}$, $R^{12}$, $R^{13}$ and $R^{14}$,** which may be identical or different, represent:
  - a hydrogen atom;
  - a halogen atom;
  - a hydroxyl group;
  - a $(C_1-C_6)$alkyl group;
  - a $(C_1-C_6)$alkoxy group;
  - a $(C_1-C_6)$alkylthio group;
  - a carboxyl group;
  - an alkyl carboxylate or alkoxycarbonyl group;
  - an optionally substituted amino group;
  - an optionally substituted linear or branched alkenyl group;
  - an optionally substituted cycloalkyl group;
  - an aryl group;
  - a substituted aryl group;
  - a group containing one or more silicon atoms;
  - a (di)((hydroxy)$(C_1-C_6)$alkyl)amino group;
  - an -O-sugar group or glycosyloxyl such as -O-glucoside;
  - a group R-Z-C(X)-Y- with R representing a hydrogen atom or a $(C_1-C_6)$alkyl or aryl group, which is optionally substituted, especially with at least one hydroxyl group, such as 3,4,5-trihydroxyphenyl; Y and Z, which may be identical or different, represent a bond, an oxygen or sulfur atom or a group -N(R')- with R' representing a hydrogen atom or a $(C_1-C_6)$alkyl group, Y possibly also representing a $(C_1-C_6)$alkylene group; X representing

an oxygen or sulfur atom, or N-R" with R" representing a hydrogen atom or a ($C_1$-$C_6$)alkyl group;

- **$R^5$, $R^6$, $R^7$, $R^8$ and $R^9$,** which may be identical or different, represent a hydrogen atom or a group chosen from a hydroxyl group, a ($C_1$-$C_6$) alkyl group or a group R-Z-C(X)-Y- as defined previously;
- or alternatively the unit **(II)** constitutes the polymeric unit of a polyphenol which will be linked to the other units of said polyphenol via positions 4, 6 or 8 of the chroman ring, in which case **$R^1$,** or **$R^3$** and **$R^6$** form a covalent bond with the other units of said polyphenol;
- or else **$R^5$** with **$R^6$** and/or **$R^7$** with **$R^8$** form, together with the carbon atom which bears them, an oxo group;
- or else **$R^8$** and **$R^9$** together form a bond;

it being understood that:

- at least two radicals chosen from **$R^1$, $R^2$ , $R^3$, $R^4$, $R^{10}$ , $R^{11}$, $R^{12}$, $R^{13}$** and **$R^{14}$,** which are contiguous, represent a hydroxyl group;
- when **$R^5$ with $R^6$** and/or **$R^7$** with **$R^8$** together form an oxo group, or **$R^8$** with **$R^9$** together form a bond, then ---- represent a single bond;
- when ---- is a double bond between the oxygen atom and the carbon atom in position 2, then **$R_9$** is absent, and when ---- is a double bond between the carbon atoms in positions 3 and 4, then **$R_5$** and **$R_7$ are** absent;
- when ---- borne by the oxygen atom is a double bond, then the compound of formula **(II)** is cationic and an organic or mineral anionic counterion such as halide is associated therewith.

Preferably, the acid is hydrochloric acid, which results in chlorides.

Preferably, the group R-Z-C(X)-Y- of the compounds of formula **(II)** represents a 3,4,5-trihydroxyphenyl-1-carbonyloxy (-O-gallate). Preferably, the radicals **$R_1$, $R_2$, $R_3$, $R_4$, $R_{10}$, $R_{11}$, $R^{12}$, $R_{13}$** and **$R_{14}$** are chosen from a hydrogen atom and hydroxyl, glycosyloxy and alkoxy groups.

More particularly, the ortho-diphenols which may be used in the invention may in particular be:

- i) the flavanols of formula **(IIa)** which comprise at least two ortho hydroxyl groups, such as catechin and epicatechin gallate, oligomers and polymers thereof known as proanthocyanidols or fused tannins such as theaflavin, theaflavin 3'-O-gallate, theaflavin 3,3'-O-digallate, proanthocyanidins A1, A2, B1, B2, B3 and C1 such as profisetinidin and procyanidin, and mixtures of the preceding compounds.

Flavan-3-ol or flavanol:

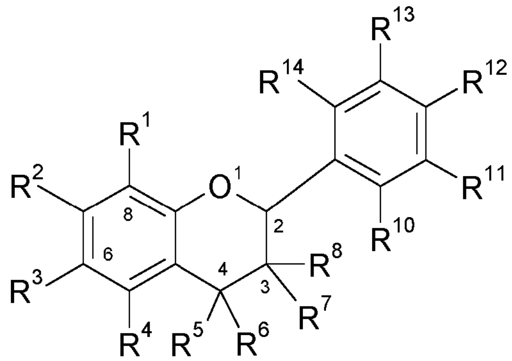

**(IIa)**

and also the organic or mineral acid or base salts thereof, the optical isomers, geometrical isomers and tautomers thereof, and the solvates thereof such as hydrates;
in which formula **(IIa) $R^7$** and **$R^8$,** which may be identical or different, represent a hydrogen atom or a group chosen from hydroxyl, ($C_1$-$C_6$)alkyl, or a group R-Z-C(X)-Y- as defined previously, preferably O-gallate, it being understood that at least one of the two groups **$R^7$** or **$R^8$** represents a hydroxyl group or R-Z-C(X)-Y- as defined previously; **$R^5$ and $R^6$,** which may be identical or different, represent a hydrogen atom or a hydroxyl group or **$R^6$** and **$R^8$** form a bond or the unit **(IIa)** constitutes the polymeric unit that will be attached to the other units via positions 4, 6 or 8, in which case **$R^1$,** or **$R^3$** and **$R^6$** form a bond; the radicals **$R^1$, $R^2$, $R^3$, $R^4$, $R^{10}$, $R^{11}$, $R^{12}$, $R^{13}$** and **$R^{14}$** being as defined previously.

According to a particular embodiment of the invention, one or more ortho-diphenol derivatives are of formula

**(IIapoly):**

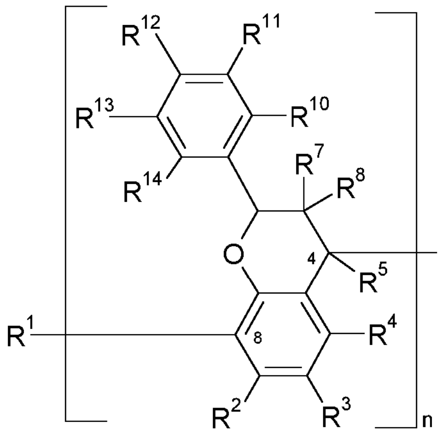

**(IIa-poly)**

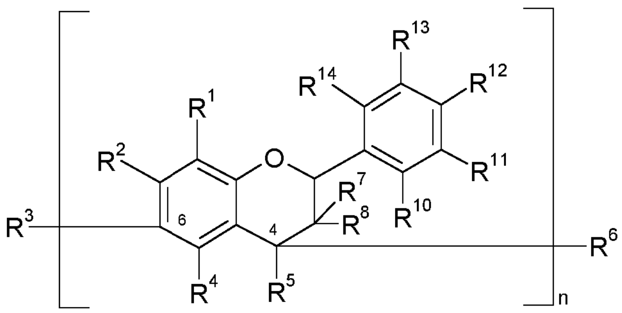

**(IIa'-poly)**

in which formulae **(IIa-poly)** and **(IIa'-poly)** the radicals $\mathbf{R^1}$, $\mathbf{R^2}$, $\mathbf{R^3}$, $\mathbf{R^4}$, $\mathbf{R^5}$, $\mathbf{R^6}$, $\mathbf{R^7}$, $\mathbf{R^8}$, $\mathbf{R^{10}}$, $\mathbf{R^{11}}$, $\mathbf{R^{12}}$, $\mathbf{R^{13}}$ and $\mathbf{R^{14}}$ are as defined previously.

n, the polymeric unit repetition which is an integer in particular between 2 and 5 inclusively, preferably equal to 3.

Among the proanthocyanidin compounds, examples that may be mentioned include profisetinidin and procyanidin, the formulae of which are represented below:

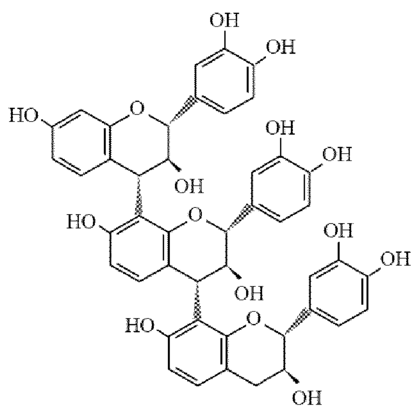

Profisetinidin

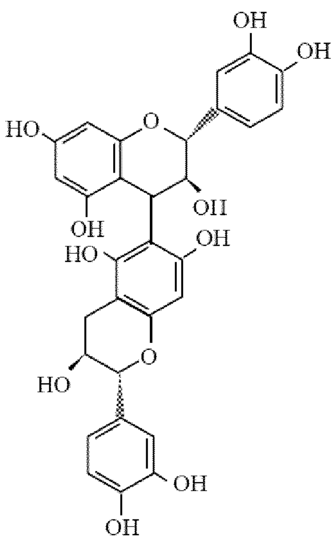

Procyanidin

- ii) the flavonols of formula **(IIb)** which comprise at least two ortho hydroxyl groups, such as quercetin, myricetol, fisetin, heterosides thereof, and methoxy derivatives thereof such as rhamnetin.

Flavonol: 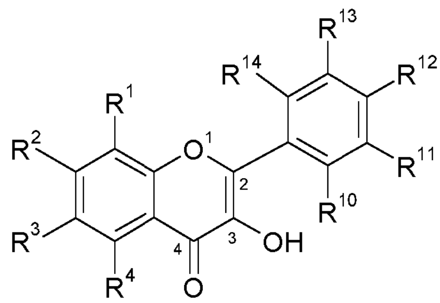 **(IIb)**

3-hydroxy-2-phenylchromen-4-one

and also the organic or mineral acid or base salts thereof, the optical, geometrical and tautomeric isomers thereof, and the solvates thereof such as hydrates;
in which formula **(IIb)** the radicals **$R^1$, $R^2$, $R^3$, $R^4$, $R^{10}$, $R^{11}$, $R^{12}$, $R^{13}$** and **$R^{14}$** are as defined previously.

- iii) the flavones of formula **(IIc)** which comprise at least two ortho hydroxyl groups, such as luteolin, and heterosides thereof such as luteolol 7-O-glucoside, baicalin and orientin.

Flavone: 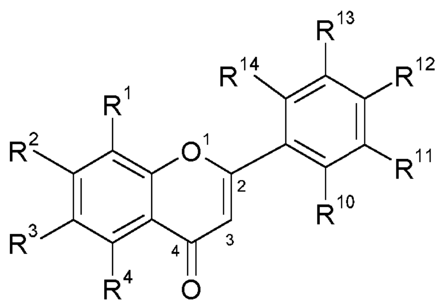 **(IIc)**

2-phenylchromen-4-one

and also the organic or mineral acid or base salts thereof, the optical, geometrical and tautomeric isomers thereof, and the solvates thereof such as hydrates;
in which formula **(IIc)** the radicals **$R^1$, $R^2$, $R^3$, $R^4$, $R^{10}$, $R^{11}$, $R^{12}$, $R^{13}$** and **$R^{14}$** are as defined previously.

- iv) the dihydroflavonols or dihydroxyflavanonols of formula **(IId)** which comprise at least two ortho hydroxyl groups, such as dihydroquercetol, and heterosides thereof such as dihydroquercetol 3-O-rhamoside.

Dihydroflavonol or Flavanonol:

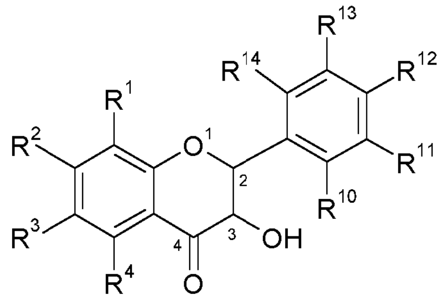

**(IId)**

3-hydroxy-2,3-dihydro-2-phenylchromen-4-one

and also the organic or mineral acid or base salts thereof, the optical, geometrical and tautomeric isomers thereof, and the solvates thereof such as hydrates;
in which formula **(IId)** the radicals **$R^1$, $R^2$, $R^3$, $R^4$, $R^{10}$, $R^{11}$, $R^{12}$, $R^{13}$** and **$R^{14}$** are as defined previously.

- v) the flavanones of formula **(IIe)** which comprise at least two ortho hydroxyl groups, such as eriodictyol.

Flavanone:

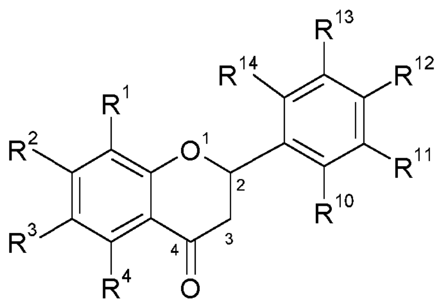

**(IIe)**

2,3-dihydro-2-phenylchromen-4-one

and also the organic or mineral acid or base salts thereof, the optical, geometrical and tautomeric isomers thereof, and the solvates thereof such as hydrates;
in which formula **(IIe)** the radicals **$R^1$, $R^2$, $R^3$, $R^4$, $R^{10}$, $R^{11}$, $R^{12}$, $R^{13}$** and **$R^{14}$** are as defined previously.

- vi) the flavan-3,4-diols or leucoanthocyanidins of formula **(IIf)** which comprise at least two ortho hydroxyl groups, such as leucocyanidol (= procyanidol).

Flavan-3,4-diol:

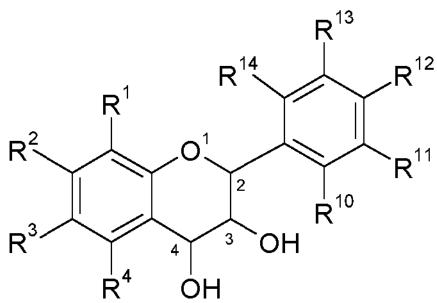

**(IIf)**

and also the organic or mineral acid or base salts thereof, the optical isomers, geometrical isomers and tautomers thereof, and the solvates thereof such as hydrates;

in which formula **(IIf)** the radicals **$R^1$, $R^2$, $R^3$, $R^4$, $R^{10}$, $R^{11}$, $R^{12}$, $R^{13}$** and **$R^{14}$** are as defined previously.

- vii) anthocyanidols (or anthocyanidins) of formula (IIg) which comprise at least two ortho hydroxyl groups, such as cyanidol, delphinidin, aurantinidin, luteolinidin, heterosides thereof, such as dihydroxyanthocyans (or dihydroxyanthocyanosides, or anthocyanins on the English model), oligomers and polymers thereof such as proanthocyanins.

Anthocyanidol or Anthocyanidin:

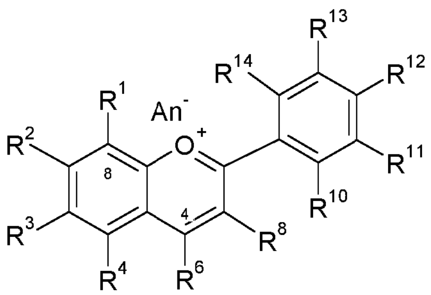

**(IIg)**

Flavylium cation

in which formula **(IIg)** the radicals **$R^1$, $R^2$, $R^3$, $R^4$, $R^6$, $R^8$, $R^{10}$, $R^{11}$, $R^{12}$, $R^{13}$** and **$R^{14}$** are as defined previously.

$An^-$ represents an organic or mineral anionic counterion, which may be present or absent. If it is absent, one of the groups $R^1$ to $R^{14}$ will be in anionic form, for example a hydroxyl group will be converted into hydroxylate $-O^-$, carboxyl converted into carboxylate $-C(O)-O^-$.

According to another particular embodiment of the invention, one or more ortho-diphenol derivatives are of formula **(IIgpoly):**

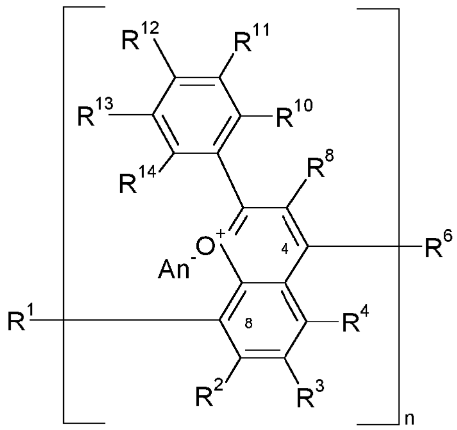

**(IIg-poly)**

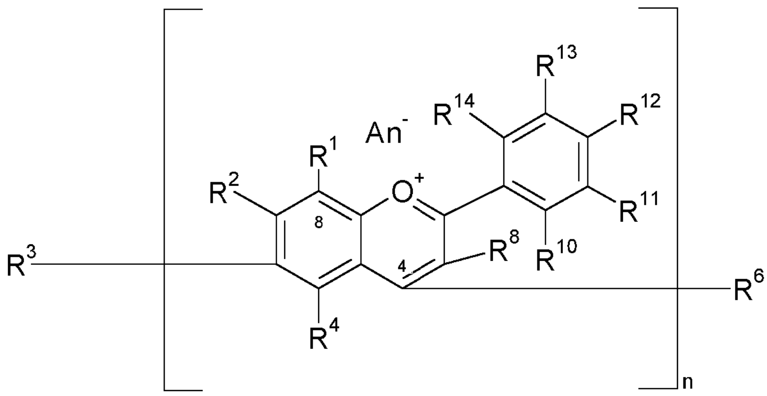

**(IIg'-poly)**

in which formulae **(IIg-poly)** and **(IIg'poly)** the radicals $R^1$, $R^2$, $R^3$, $R^4$, $R^6$, $R^8$, $R^{10}$, $R^{11}$, $R^{12}$, $R^{13}$ and $R^{14}$ are as defined previously.

In one variant of the invention, the ortho-diphenol derivatives according to the invention are proanthocyanidin derivatives such as profisetinidin and procyanidin.

According to another particular embodiment of the invention, the composition comprises as ortho-diphenol(s) one or more neoflavanol or neoflavanone derivatives or mixtures thereof; preferentially, neoflavonol derivatives.

More particularly, the neoflavanols and neoflavanones are of formulae **(III)** and **(IV)** and tautomeric forms thereof, stereoisomers thereof, addition salts thereof with a cosmetically acceptable acid or base, and also the hydrates;

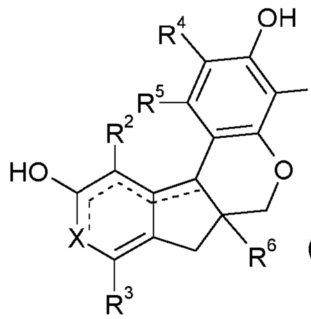

**(III)**

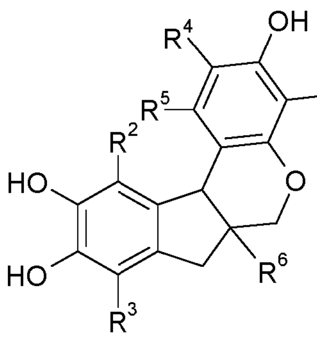

**(IV)**

in which formulae **(III)** and **(IV):**

- ---- represents a conjugated double or single carbon-carbon bond,
- X represents a group:

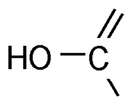

or

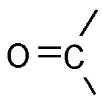

- **$R^1$, $R^2$, $R^3$, $R^4$, $R^5$ and $R^6$**, which may be identical or different, represent a hydrogen atom, a hydroxyl group, an optionally substituted alkyl group, an optionally substituted alkoxy group or an optionally substituted acyloxy group.

Preferably, the acid is hydrochloric acid, which results in chlorides.

The compounds of formula **(III)** as defined previously may be in two tautomeric forms denoted **(IIIa)** and **(IIIb):**

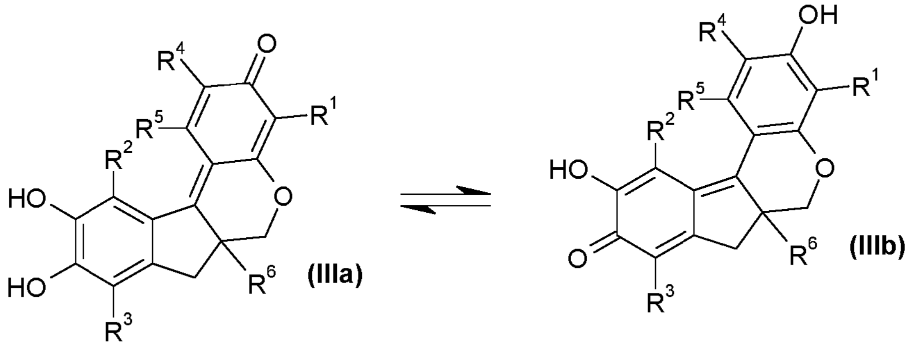

(IIIa) (IIIb)

According to a particular embodiment of the invention, the compounds of formula **(III)** or **(IV)** comprise a radical $R^6$ which represents a hydroxyl group.

Another particular embodiment of the invention relates to the compounds of formula **(III)** or **(IV)** for which $R^1$ represents a hydrogen atom or a hydroxyl group.

In another variant of the invention, the ortho-diphenol derivatives according to the invention are compounds of formula **(α),** or an oligomer thereof, in salified or non-salified form:

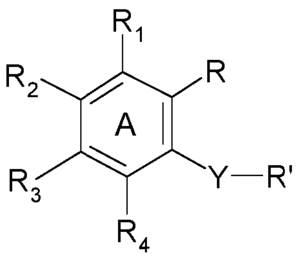

(α)

in which formula **(α):**

- **R** represents a hydrogen atom or a hydroxyl group;
- **R'** represents a hydroxyl group or the phenoxy group according to **(α'):**

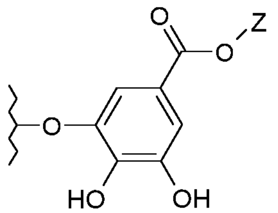

- **Y** represents a divalent, trivalent or tetravalent group chosen from $(C_1-C_6)$alkylene such as methylene; carbonyl -C(O)-, -CH=, >C<;
- **$R_1$, $R_2$ and $R_3$,** which may be identical or different, represent a hydrogen atom or a hydroxyl group or $(C_1-C_6)$alkoxy such as methoxy;
- **$R_4$** represents a hydrogen atom, a hydroxyl group or -O-glycoside; or alternatively the radicals **R,** Y and **R'** form, together with the carbon atoms that bear them, a heterocyclic group fused to ring A, of formula **(β), (γ)** or **(γ')** below:

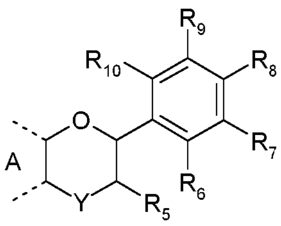

(β)

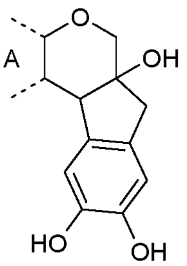

(γ)

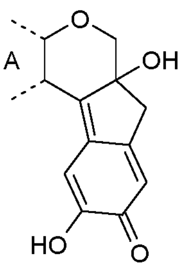

(γ')

- **$R_5$** represents a hydrogen atom or a hydroxyl or -O-glycoside group;
- **$R_6$** to **$R_{10}$**, which may be identical or different, represent a hydrogen atom or a hydroxyl group, preferably **$R_6$** and **$R_{10}$** represent a hydrogen atom, **$R_7$, $R_8$** and **$R_9$** represent a hydrogen atom or a hydroxyl group;
- **Z** represents a hydrogen atom or the group **(**δ**)** below:

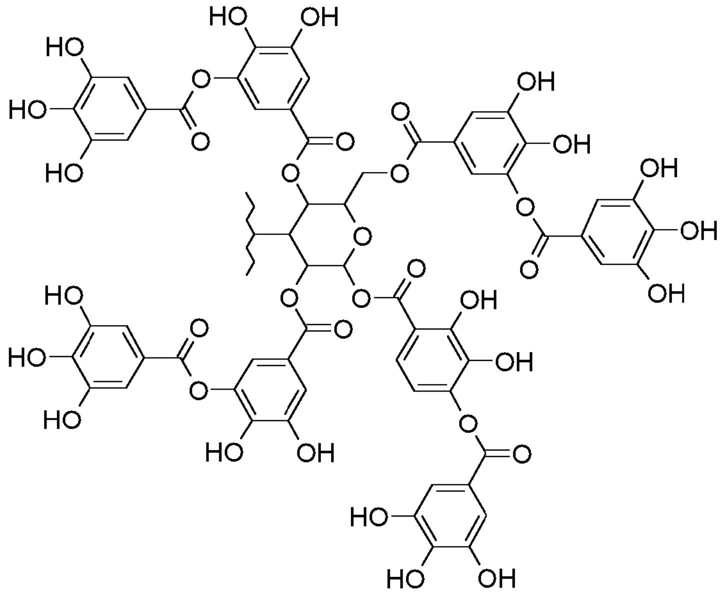

it being understood that the compound of formula **($\alpha$)** bears at least two hydroxyl groups ortho to a phenyl radical.

In a preferred variant, the ortho-diphenol derivatives according to the invention are compounds of formula **($\alpha$),** and more particularly polyphenols such as tannic acid.

The ortho-diphenol derivatives according to the invention may in particular be a mixture of proanthocyanidin derivatives such as profisetinidin and procyanidin and of compounds of formula **($\alpha$),** and more particularly polyphenols such as tannic acid.

When the dye precursors have D and L forms, the two forms may be used in the compositions according to the invention, as may the racemic mixtures.

Among the extracts of tree wood, mention may be made of extracts of pine bark and extracts of campeachy wood or extracts of quebracho wood.

Use may also be made of mixtures of plant extracts.

According to a particular embodiment of the invention, the ortho-diphenol derivative(s) are natural extracts, rich in ortho-diphenols. According to a preferred form, the ortho-diphenol derivative(s) are solely natural extracts.

The natural extracts according to the invention may be in the form of powders or liquids. Preferably, the extracts of the invention are in the form of powders.

According to the invention, the synthetic, natural ortho-diphenol derivative(s), and/or the natural extract(s) used as compound in the composition according to the invention represent a content between 5% to 10 % by weight relative to the total weight of the composition.

**Alkali metal or alkaline-earth metal (bi)carbonate:**

The composition according to the invention comprises at least one alkali metal or alkaline-earth metal (bi)carbonate compound.

The term "alkali metal or alkaline-earth metal (bi)carbonate" means:

a) alkali metal carbonates ($Met^{2+}$, $CO_3^{2-}$), alkaline-earth metal carbonates ($Met'^{2+}$, $CO_3^{2-}$) with Met' representing an alkaline-earth metal and Met representing an alkali metal, and

b) bicarbonates, also known as hydrogen carbonates, of the following formulae:

- $R'^{+}$, $HCO_3^-$ with R' representing a hydrogen atom or an alkali metal, and when R' represents a hydrogen atom, the hydrogen carbonate is then known as a dihydrogen carbonate ($CO_2$, $H_2O$); and
- Met'2$^{+}$ $(HCO_3^-)_2$, with Met' representing an alkaline-earth metal.

Mention may be made of Na, K, Mg and Ca carbonates or hydrogen carbonates and mixtures thereof, and in particular sodium hydrogen carbonate. These hydrogen carbonates may originate from a natural water, for example spring water from the Vichy basin or from La Roche Posay or Badoit water (cf. for example, patent FR 2 814 943). Particularly, mention may be made of sodium carbonate [497-19-8] = Na2CO3, sodium hydrogen carbonate or sodium

bicarbonate [144-55-8] = $NaHCO3$, and sodium dihydrogen carbonate = $Na(HCO3)2$.

Preferably, the alkali metal or alkaline-earth metal (bi)carbonate compound is sodium bicarbonate.

According to the invention, the alkali metal or alkaline-earth metal (bi)carbonate compound(s) represent from 0.001% to 20% by weight, preferably from 0.001% to 15% by weight and better still from 0.1% to 10% by weight relative to the total weight of the composition.

**Water-soluble organic solvent:**

The composition according to the invention comprises at least one water-soluble organic solvent in a content of greater than or equal to 10% by weight relative to the weight of the composition.

For the purposes of the present invention, the term "water-soluble solvent" means a compound that is liquid at ordinary temperature (25°C) and at atmospheric pressure (760 mmHg; i.e. $1.013\times10^5$ Pa), with a water solubility under these conditions of greater than or equal to 5%.

The water-soluble organic solvent is a non-aromatic $C_2$-$C_6$ alcohol, preferably a non-aromatic $C_2$-$C_6$ monoalcohol and more preferentially ethyl alcohol.

The water-soluble organic solvent(s) represent(s) from 10% to 50% by weight, preferably between 10% and 40% by weight, and even more preferentially from 20% to 40% by weight, relative to the total weight of the composition.

**Reducing agent:**

The composition according to the invention comprises at least one reducing agent.

The reducing agent is chosen from alkali metal or alkaline-earth metal sulfites or bisulfites. As sulfites and bisulfites that may be used, mention is made of alkali metal, alkaline-earth metal, ammonium or alkanolamine sulfites or bisulfites, and in particular sodium or potassium sulfite or bisulfite, and monoethanolamine sulfite or bisulfite. Preferably, the reducing agent is sodium sulfite.

The reducing agent generally represents from 0.01% to 50% by weight and preferably from 0.05% to 15% by weight relative to the total weight of the composition.

**Aqueous phase:**

The composition according to the invention comprises an aqueous phase.

The aqueous phase comprises water and the water-soluble organic solvent(s) described previously.

Preferably, water is present in a content ranging from 60% to 90% by weight, preferably from 70% to 90% by weight relative to the total weight of the composition.

**Adjuvants:**

The composition in accordance with the invention may also contain various adjuvants, such as anionic, cationic, nonionic, amphoteric or zwitterionic surfactants or mixtures thereof, anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrants, sequestrants, fragrances, buffers, dispersants, conditioning agents, for instance volatile or non-volatile, modified or unmodified silicones, film-forming agents, ceramides, preserving agents and opacifiers.

Said adjuvants are preferably chosen from surfactants such as anionic or nonionic surfactants or mixtures thereof and mineral or organic thickeners.

The composition in accordance with the invention may also contain an organic thickening polymer.

These organic thickening polymers are preferably chosen from cellulose-based thickeners (hydroxyethylcellulose, hydroxypropylcellulose or carboxymethylcellulose), guar gum and its derivatives (hydroxypropyl guar), gums of microbial origin (xanthan gum, scleroglucan gum), optionally crosslinked homopolymers or copolymers of acrylic acid or of acrylamidopropanesulfonic acid, in particular copolymers of acrylamide and of acrylamidopropanesulfonic acid such as Sepigel 305 sold by SEPPIC and associative polymers (polymers comprising hydrophilic regions and hydrophobic regions having a fatty chain (alkyl or alkenyl chain comprising at least 10 carbon atoms) which are capable, in an aqueous medium, of reversibly associating with one another or with other molecules).

The composition in accordance with the invention may also contain one or more fatty substances.

The fatty substances are preferably chosen from C6-C16 hydrocarbons, hydrocarbons containing more than 16 carbon atoms, non-silicone oils of animal origin, triglycerides of plant or synthetic origin, fluoro oils, fatty alcohols, non-salified fatty acids, esters of fatty acid and/or of fatty alcohol other than triglycerides and non-silicone waxes, in particular from plants, non-silicone waxes, and silicones, and mixtures thereof, more preferentially from liquid paraffin

or liquid petroleum jelly.

The above adjuvants are generally present in an amount for each of them of between 0.01% and 40% by weight relative to the weight of the composition, and preferably between 0.1% and 20% by weight relative to the weight of the composition.

Needless to say, a person skilled in the art will take care to select this or these optional additional compound(s) such that the advantageous properties intrinsically associated with the composition in accordance with the invention are not, or are not substantially, adversely affected by the envisaged addition(s).

Additional dyes:

The composition according to the invention may comprise one or more additional direct dyes. These direct dyes are chosen, for example, from those conventionally used in direct dyeing, and among which mention may be made of any commonly used aromatic and/or non-aromatic dyes such as neutral, acidic or cationic nitrobenzene direct dyes, neutral, acidic or cationic azo direct dyes, natural direct dyes other than the ortho-diphenols as defined previously, neutral, acidic or cationic quinone and in particular anthraquinone direct dyes, azine, triarylmethane, indoamine, methine, styryl, porphyrin, metalloporphyrin, phthalocyanin, cyanin such as methine cyanin, streptocyanin, hemicyanin and carbocyanin direct dyes, and fluorescent dyes.

Among the natural direct dyes, mention may be made of lawsone, juglone, indigo, isatin, curcumin, spinulosin, apigenidin and orceins. Use may also be made of extracts or decoctions comprising these natural dyes and in particular henna-based extracts or poultices.

According to the invention, the additional direct dye(s) used in the composition as defined previously preferably represent from 0.001% to 10% by weight approximately of the total weight of the composition(s) and even more preferentially from 0.05% to 5% by weight approximately.

The composition according to the invention may also contain one or more oxidation bases and/or one or more couplers conventionally used for the dyeing of keratin fibres.

Among the oxidation bases, mention may be made of para-phenylenediamines, bis(phenyl)alkylenediamines, para-aminophenols, bis-para-aminophenols, ortho-aminophenols and heterocyclic bases, and the addition salts thereof.

Among these couplers, mention may be made especially of meta-phenylenediamines, meta-aminophenols, meta-diphenols, naphthalene-based couplers and heterocyclic couplers, and the addition salts thereof.

The oxidation base(s) present in said composition are generally each present in an amount of between 0.001% and 10% by weight relative to the total weight of the composition containing them.

The composition according to the invention may be in various galenical forms, such as a powder, a lotion, a mousse, a cream or a gel, or in any other form that is suitable for dyeing keratin fibres. They may also be packaged in a propellant-free pump-action bottle or under pressure in an aerosol container in the presence of a propellant and form a foam.

According to a particular embodiment of the invention, the pH of the composition according to the invention is greater than or equal to 5 and preferably ranges from 5 to 10.

The pH of the composition according to the invention may be adjusted to the desired value by means of acidifying or basifying agents usually used in the dyeing of keratin fibres, or alternatively using standard buffer systems.

Among the acidifying agents for the compositions used in the invention, examples that may be mentioned include mineral or organic acids, for instance hydrochloric acid, orthophosphoric acid, sulfuric acid, carboxylic acids, for instance acetic acid, tartaric acid, citric acid or lactic acid, or sulfonic acids.

The basifying agents may be chosen from aqueous ammonia, alkali metal carbonates or bicarbonates such as sodium carbonate or bicarbonate, potassium carbonate or bicarbonate, sodium hydroxide or potassium hydroxide, or mixtures thereof.

**Dyeing process:**

A subject of the invention is also a process for dyeing keratin fibres, in particular human keratin fibres such as the hair, comprising the application to the keratin fibres of the composition as defined previously.

At the time of use, the composition as described above is applied to the keratin fibres and left on for approximately 3 to 50 minutes, preferably approximately 5 to 30 minutes, then a rinsing and shampooing step may follow, then another rinsing, and finally drying. The composition may be applied one after the other, in any order, with or without intermediate rinsing.

The composition may be applied with or without final rinsing, optionally in combination with another formula.

The keratin fibres may be dried at a temperature ranging from 50 to 80°C or left to dry in the open air.

The keratin fibres may be dried by means of a hairdryer, a drying hood or a straightening iron so as to carry out a hair shaping step.

A subject of the invention is also a process for dyeing keratin fibres, in particular human keratin fibres such as the hair, comprising the application to the keratin fibres of a first dye composition comprising at least one ortho-diphenol comprising at least one aromatic ring and comprising at least two hydroxyl groups (OH) borne by two adjacent carbon atoms of the aromatic ring, the content of ortho-diphenol being between 5% and 10% by weight relative to the total weight of the composition, said ortho-diphenol being chosen from natural ortho-diphenol chosen from extracts of tree wood, at least one alkali metal or alkaline-earth metal (bi)carbonate compound the alkali metal or alkaline-earth metal (bi)carbonate compound(s) representing from 0.001% to 20% by weight relative to the total weight of the composition, at least one water-soluble organic solvent the water-soluble organic solvent(s) representing from 10% to 50% by weight relative to the total weight of the composition, said solvent being chosen from non-aromatic $C_2$-$C_6$ alcohol and at least one reducing agent, the reducing agent representing from 0.01% to 50% by weight relative to the total weight of the composition, the reducing agent being chosen from alkali metal or alkaline-earth metal sulfites or bisulfites and then of a second composition comprising at least one metal salt.

The metal salt(s) used in the process of the invention are preferably salts of metals from columns 4 to 12 of the Periodic Table of the Elements.

Particularly, the metal salts are chosen from manganese (Mn) and zinc (Zn) salts.

For the purposes of the present invention, the term "salts" means the oxides of these metals and salts per se derived in particular from the action of an acid on a metal. Preferably, the salts are not oxides. Among the salts, mention may be made of halides, such as chlorides, fluorides and iodides; sulfates, phosphates; nitrates; perchlorates and carboxylic acid salts and polymer salts, and also mixtures thereof.

The carboxylic acid salts that may be used in the invention also include salts of hydroxylated carboxylic acids such as gluconate.

As examples of polymer salts, mention may be made of manganese pyrrolidonecarboxylate.

By way of example, mention may be made of manganese chloride, manganese fluoride, manganese acetate tetrahydrate, manganese lactate trihydrate, manganese phosphate, manganese iodide, manganese nitrate trihydrate, manganese bromide, manganese perchlorate tetrahydrate, manganese sulfate monohydrate and manganese gluconate. The salts advantageously used are manganese gluconate and manganese chloride.

Among the zinc salts, mention may be made of zinc sulfate, zinc gluconate, zinc chloride, zinc lactate, zinc acetate, zinc glycinate and zinc aspartate.

The manganese and zinc salts may be introduced in solid form into the compositions or may be derived from a natural, mineral or thermal water that is rich in these ions or alternatively from seawater (especially the Dead Sea). They may also originate from mineral compounds, for instance earths, ochres such as clays (for example green clay) or even from a plant extract containing them (cf. for example patent FR 2 814 943).

According to one preferred embodiment of the invention, the metal salts used represent from 0.001% to 0.1% by weight approximately relative to the total weight of the composition(s) containing this or these metal salts, and even more preferentially from 0.05% to 10% by weight approximately.

Particularly, the metal salts of the invention are in oxidation state II, such as Mn (II) and Zn (II). Preferentially, the metal salts are manganese salts.

According to one embodiment of the invention, the first composition as defined previously is applied to wet or dry keratin fibres. Next, the second composition comprising the metal salts as defined previously is applied.

The keratin fibre dyeing process optionally comprises a rinsing step, and/or a drying and/or draining step after the application of the first composition and before the application of the second composition.

The leave-on time of the first composition on the keratin fibres may range from 1 to 60 minutes and is preferably from 5 minutes to 20 minutes.

According to a particular embodiment variant, the temperature during the two steps of the process of the invention is conventionally between room temperature (between 15 and 25°C) and 180°C if a straightening iron is used after application of the first composition and of the second composition, preferably between room temperature and 80°C, preferably between room temperature and 60°C, better still from room temperature to 40°C. The two steps of the process of the invention may be performed at different temperatures.

In this particular embodiment, the first composition is applied to the keratin fibres and is left on for 5 to 10 minutes.

After rinsing the first composition, the fibres are drained dry with a towel.

The leave-on time of the second composition on the keratin fibres may range in general from about 1 minute to 60 minutes and preferably from 10 minutes to 30 minutes.

After applying the second composition as defined previously, the human keratin fibres are preferably rinsed with water. They may be washed with a shampoo, followed by rinsing with water, before being dried at a temperature ranging from 50 to 80°C or left to dry in the open air.

The keratin fibres may be dried by means of a hairdryer, a drying hood or a straightening iron so as to perform a hair shaping step.

The examples that follow serve to illustrate the invention without, however, being limiting in nature.

## EXAMPLES

### Example 1 :

The following compositions were prepared (unless otherwise mentioned, the amounts are expressed in g% of product per se):

| Ingredients | Invention A1 | Comparative A2 |
|---|---|---|
| Quebracho extract | x | x |
| Sodium bicarbonate | 5 | 5 |
| Sodium sulfite | 0.1 | 0.1 |
| Ethanol | 10 | 7 |
| Water | qs 100 | qs 100 |

Results:

The stability of the formulations was evaluated macroscopically (with the naked eye) after 48 hours.
The following results are obtained:

| Content of quebracho extract | x= 5 g% | x=7 g% | x=10 g% |
|---|---|---|---|
| Evaluation of comparative composition A2 | Deposits | Deposits | Deposits |
| Evaluation of composition A1 according to the invention | Homogeneous | Homogeneous | Homogeneous |

Thus, the composition according to the invention comprising a water-soluble organic solvent in a content of greater than or equal to 10% by weight relative to the total weight of the composition has good stability relative to the composition according to the invention comprising a water-soluble organic solvent in a content equal to 7 g%.

### Example 2

The following compositions were prepared (unless otherwise mentioned, the amounts are expressed in g% of product per se):

| | A Comparative | A' Invention | B Comparative | B' Invention |
|---|---|---|---|---|
| Quebracho extract (74.6% Polyphenols) | 1am | 6am | | |
| Pine extract 67% (Polyphenols) | | | 1am | 6am |
| Sodium bicarbonate | 5 | 5 | 5 | 5 |
| Sodium sulfite | 0.1 | 0.1 | 0.1 | 0.1 |
| Ethanol | 10 | 10 | 10 | 10 |
| Water | QSP 100 | QSP 100 | QSP 100 | QSP 100 |
| *am = active material | | | | |

### Composition C (oxidizing agent)

| | |
|---|---|
| 50% hydrogen peroxide solution | 2.4 |
| Cetylstearyl alcohol (30/70 C16/C18) | 1.5 |
| BEHENTRIMONIUM CHLORIDE at 79% in water | 1 |

(continued)

| | |
|---|---|
| AMODIMETHICONE (and) TRIDECETH-6 (and) CETRIMONIUM CHLORIDE (XIAMETER MEM-8299 EMULSION , Dow Corning) | 1 |
| Deionized water | qs 100 |

**Composition D**

| | |
|---|---|
| LAURETH-5 CARBOXYLIC ACID (4.5 EO) at 90% in water | 6.67 |
| Cocamidopropyl betaine at 38% in aqueous solution | 18.42 |
| Oxyethylenated lauric alcohol (4 EO) | 1 |
| Oxyethylenated lauric alcohol (12 EO) | 4 |
| Sodium lauryl ether sulfate (2 EO) at 70% in aqueous solution | 10 |
| Sodium chloride | 1 |
| Sodium benzoate | 0.5 |
| Salicylic acid | 0.2 |
| Polydimethyldiallylammonium chloride at 40% in water, non-stabilized | 1.25 |
| Manganese gluconate (CAS No. 6485-39-8) | 0.05 |
| POLYQUATERNIUM-53 | 1.25 |
| Water | qs 100 g |

Composition D is applied on locks of natural hair comprising 90% of white hairs (2g of formulation per 1 g of hair) with 5 massages and rinsed.

Compositions A, A', B, B 'are mixed with the developer C in a 1:1 ratio and the mixtures are applied on the hair (2g of formulation per 1 g of hair) and let 10 minutes at room temperature.

Colorimetric results

The coloring of the hair is evaluated visually and read on a Minolta Spectrophotometer (CM2600d, illuminant D65, angle 10°, SCI values) for the L*, a*, b* colorimetric measurements.

In this L*, a*, b* system, L* represents the intensity of the color, a* indicates the green/red color axis and b* indicates the blue/yellow color axis. The lower the value of L, the darker or more intense the color. The higher the value of a*, the redder the shade; the higher the value of b*, the yellower the shade.

The color variation between treated and non treated locks of white hairs ΔE* represents the color uptake and is calculated using the following formula :

$$\Delta E^* = \sqrt{(L^* - L_o^*)^2 + (a^* - a_o^*)^2 + (b^* - b_o^*)^2}$$

In which L*, a* and b* represent the values measured after coloration of natural hair comprising 90% of white hairs and L*0, a0* et b0* represent the values measured before coloration of natural hair comprising 90% of white hairs.

The highest is the ΔE value, the most important is the difference of color between colored and non colored hair locks, i.e. the most important is the color uptake.

| | **L*(D65)** | **a*(D65)** | **b*(D65)** | **ΔE*ab(D65)** |
|---|---|---|---|---|
| Non treated hair | 69,41 | 0,74 | 16,11 | - |
| A Comp | 60,17 | 8,86 | 17,2 | 12,35 |

(continued)

| | L*(D65) | a*(D65) | b*(D65) | ΔE*ab(D65) |
|---|---|---|---|---|
| A'Inv | 43,18 | 15,38 | 17,24 | 30,06 |
| B Comp | 64,39 | 8,35 | 21,78 | 10,73 |
| B' Inv | 52,78 | 15,02 | 23,83 | 23,24 |

These results show that inventive composition A' and B' exhibit an improved colored uptake compared with comparative compositions A and B.

Furthermore, the color intensity is more important with compositions A' and B'.

## Example 3

The following compositions were prepared (unless otherwise mentioned, the amounts are expressed in g% of product per se):

| | E Comparative | E' Invention |
|---|---|---|
| Quebracho extract (74.6% Polyphenols) | 6 am | 6 am |
| Sodium bicarbonate | 5 | 5 |
| Sodium sulfite | 0.1 | 0.1 |
| Dipropylene glycol | 7 | 10 |
| Water | QSP 100 | QSP 100 |

The stability of the composition is visually assessed after 24h : the comparative composition E presents deposits and a film on its surface, the comparative composition E' is completely stable .

## Claims

**1.** Composition comprising:

- at least one ortho-diphenol comprising at least one aromatic ring and comprising at least two hydroxyl groups (OH) borne by two adjacent carbon atoms of the aromatic ring, the content of ortho-diphenol being between 5% and 10% by weight relative to the total weight of the composition, said ortho-diphenol being chosen from natural ortho-diphenol chosen from extracts of tree wood,
- at least one alkali metal or alkaline-earth metal (bi)carbonate compound, the alkali metal or alkaline-earth metal (bi)carbonate compound(s) representing from 0.001% to 20% by weight relative to the total weight of the composition,
- at least one water-soluble organic solvent, the water-soluble organic solvent(s) representing from 10% to 50% by weight relative to the total weight of the composition, said solvent being chosen from non-aromatic $C_2$-$C_6$ alcohol and
- at least one reducing agent, the reducing agent representing from 0.01% to 50% by weight relative to the total weight of the composition, the reducing agent being chosen from alkali metal or alkaline-earth metal sulfites or bisulfites.

**2.** Composition according to Claim 1, **characterized in that** the natural ortho-diphenol(s) are chosen from extracts of pine bark, extracts of campeachy wood or extracts of quebracho wood, and mixtures thereof.

**3.** Composition according to any one of the preceding claims, **characterized in that** the alkali metal or alkaline-earth metal (bi)carbonate compound is sodium bicarbonate.

**4.** Composition according to any one of the preceding claims, **characterized in that** the alkali metal or alkaline-earth metal (bi)carbonate compound(s) represent from 0.01% to 15% by weight and better still from 0.1% to 10% by

weight relative to the total weight of the composition.

5. Composition according to any one of the preceding claims, **characterized in that** the water-soluble organic solvent is a non-aromatic $C_2$-$C_6$ monoalcohol and more preferentially ethyl alcohol.

6. Composition according to any one of the preceding claims, **characterized in that** the water-soluble organic solvent(s) represent from 10% and 40% by weight and even more preferentially from 20% to 40% by weight, relative to the total weight of the composition.

7. Composition according to any one of the preceding claims, **characterized in that** the reducing agent is sodium sulfite.

8. Composition according to any one of the preceding claims, **characterized in that** the reducing agent represents from 0.05% to 15% by weight relative to the total weight of the composition.

9. Process for dyeing keratin fibres, in particular human keratin fibres such as the hair, **characterized in that** a composition as defined according to any one of Claims 1 to 8 is applied to the keratin fibres.

10. Process for dyeing keratin fibres, in particular human keratin fibres such as the hair, **characterized in that** a first composition as defined according to any one of Claims 1 to 8 is applied to the keratin fibres, followed by a second composition comprising at least one metal salt.

## Patentansprüche

1. Zusammensetzung, umfassend:

   - mindestens ein ortho-Diphenol mit mindestens einem aromatischen Ring und mindestens zwei Hydroxylgruppen (OH) an zwei benachbarten Kohlenstoffatomen des aromatischen Rings, wobei der Gehalt an ortho-Diphenol zwischen 5 und 10 Gew.-%, bezogen auf das Gesamtgewicht der Zusammensetzung, liegt, wobei das ortho-Diphenol aus natürlichem ortho-Diphenol, das aus Baumholzextrakten ausgewählt ist, ausgewählt ist,
   - mindestens eine Alkalimetall- oder Erdalkalimetall(hydrogen)carbonat-Verbindung, wobei die Alkalimetall- oder Erdalkalimetall(hydrogen)carbonat-Verbindung bzw. Alkalimetall- oder Erdalkalimetall-(hydrogen)carbonat-Verbindungen 0,001 bis 20 Gew.-%, bezogen auf das Gesamtgewicht der Zusammensetzung, ausmacht bzw. ausmachen,
   - mindestens ein wasserlösliches organisches Lösungsmittel, wobei das wasserlösliche organische Lösungsmittel bzw. die wasserlöslichen organischen Lösungsmittel 10 bis 50 Gew.-%, bezogen auf das Gesamtgewicht der Zusammensetzung, ausmacht bzw. ausmachen, wobei das Lösungsmittel aus nichtaromatischen $C_2$-$C_6$-Alkoholen ausgewählt ist, und
   - mindestens ein Reduktionsmittel, wobei das Reduktionsmittel 0,01 bis 50 Gew.-%, bezogen auf das Gesamtgewicht der Zusammensetzung, ausmacht, wobei das Reduktionsmittel aus Alkalimetall- oder Erdalkalimetallsulfiten oder -hydrogensulfiten ausgewählt ist.

2. Zusammensetzung nach Anspruch 1, **dadurch gekennzeichnet, dass** das natürliche ortho-Diphenol bzw. die natürlichen ortho-Diphenole aus Kiefernrindenextrakten, Campecheholzextrakten oder Quebrachoholzextrakten und Mischungen davon ausgewählt ist bzw. sind.

3. Zusammensetzung nach einem der vorhergehenden Ansprüche, **dadurch gekennzeichnet, dass** es sich bei der Alkalimetall- oder Erdalkalimetall(hydrogen)carbonat-Verbindung um Natriumhydrogencarbonat handelt.

4. Zusammensetzung nach einem der vorhergehenden Ansprüche, **dadurch gekennzeichnet, dass** die Alkalimetall- oder Erdalkalimetall(hydrogen)carbonat-Verbindung bzw. Alkalimetall- oder Erdalkalimetall-(hydrogen)carbonat-Verbindungen 0,01 bis 15 Gew.-% und noch besser 0,1 bis 10 Gew.-%, bezogen auf das Gesamtgewicht der Zusammensetzung, ausmacht bzw. ausmachen.

5. Zusammensetzung nach einem der vorhergehenden Ansprüche, **dadurch gekennzeichnet, dass** es sich bei dem wasserlöslichen organischen Lösungsmittel um einen $C_2$-$C_6$-Monoalkohol und weiter bevorzugt Ethylalkohol handelt.

6. Zusammensetzung nach einem der vorhergehenden Ansprüche, **dadurch gekennzeichnet, dass** das wasserlösliche organische Lösungsmittel bzw. die wasserlöslichen organischen Lösungsmittel 10 bis 40 Gew.-% und noch weiter bevorzugt 20 bis 40 Gew.-%, bezogen auf das Gesamtgewicht der Zusammensetzung, ausmacht bzw. ausmachen.

7. Zusammensetzung nach einem der vorhergehenden Ansprüche, **dadurch gekennzeichnet, dass** es sich bei dem Reduktionsmittel um Natriumsulfit handelt.

8. Zusammensetzung nach einem der vorhergehenden Ansprüche, **dadurch gekennzeichnet, dass** das Reduktionsmittel 0,05 bis 15 Gew.-%, bezogen auf das Gesamtgewicht der Zusammensetzung, ausmacht.

9. Verfahren zum Färben von Keratinfasern, insbesondere menschlichen Keratinfasern wie dem Haar, **dadurch gekennzeichnet, dass** man auf die Keratinfasern eine Zusammensetzung gemäß einem der Ansprüche 1 bis 8 aufbringt.

10. Verfahren zum Färben von Keratinfasern, insbesondere menschlichen Keratinfasern wie dem Haar, **dadurch gekennzeichnet, dass** man auf die Keratinfasern eine erste Zusammensetzung gemäß einem der Ansprüche 1 bis 8 und anschließend eine zweite Zusammensetzung, die mindestens ein Metallsalz umfasst, aufbringt.

**Revendications**

1. Composition comprenant :

- au moins un ortho-diphénol comprenant au moins un cycle aromatique et comprenant au moins deux groupes hydroxyle (OH) portés par deux atomes de carbone adjacents du cycle aromatique, la teneur en ortho-diphénol étant comprise entre 5 % et 10 % en poids par rapport au poids total de la composition, ledit ortho-diphénol étant choisi parmi un ortho-diphénol naturel choisi parmi des extraits de bois d'arbre,
- au moins un composé de type (bi)carbonate de métal alcalin ou de métal alcalino-terreux, le(s) composé(s) de type (bi)carbonate de métal alcalin ou de métal alcalino-terreux représentant de 0,001 % à 20 % en poids par rapport au poids total de la composition,
- au moins un solvant organique soluble dans l'eau, le(s) solvant(s) organique(s) soluble(s) dans l'eau représentant de 10 % à 50 % en poids par rapport au poids total de la composition, ledit solvant étant choisi parmi un alcool non aromatique en $C_{2}$-$_{6}$ et
- au moins un agent de réduction, l'agent de réduction représentant de 0,01 % à 50 % en poids par rapport au poids total de la composition, l'agent de réduction étant choisi parmi des sulfites et des bisulfites de métal alcalin ou de métal alcalino-terreux.

2. Composition selon la revendication 1, **caractérisée en ce que** l'ortho-diphénol ou les ortho-diphénols naturel(s) est/sont choisi (s) parmi des extraits d'écorce de pin, des extraits de bois de campêche et des extraits de bois de quebracho, et des mélanges correspondants.

3. Composition selon l'une quelconque des revendications précédentes, **caractérisée en ce que** le composé de type (bi)carbonate de métal alcalin ou de métal alcalino-terreux est le bicarbonate de sodium.

4. Composition selon l'une quelconque des revendications précédentes, **caractérisée en ce que** le(s) composé(s) de type (bi)carbonate de métal alcalin ou de métal alcalino-terreux représente(nt) de 0,01 % à 15 % en poids et encore mieux de 0,1 % à 10 % en poids par rapport au poids total de la composition.

5. Composition selon l'une quelconque des revendications précédentes, **caractérisée en ce que** le solvant organique soluble dans l'eau est un monoalcool non aromatique en $C_{2-6}$ et plus préférentiellement l'alcool éthylique.

6. Composition selon l'une quelconque des revendications précédentes, **caractérisée en ce que** le(s) solvant(s) organique(s) soluble(s) dans l'eau représente (nt) de 10 % à 40 % en poids et encore plus préférentiellement de 20 % à 40 % en poids, par rapport au poids total de la composition.

7. Composition selon l'une quelconque des revendications précédentes, **caractérisée en ce que** l'agent de réduction est le sulfite de sodium.

8. Composition selon l'une quelconque des revendications précédentes, **caractérisée en ce que** l'agent de réduction représente de 0,05 % à 15 % en poids par rapport au poids total de la composition.

9. Procédé pour la coloration de fibres de kératine, en particulier de fibres de kératine humaine telles que les cheveux, **caractérisé en ce qu'**une composition telle que définie selon l'une quelconque des revendications 1 à 8 est appliquée aux fibres de kératine.

10. Procédé pour la coloration de fibres de kératine, en particulier de fibres de kératine humaine telles que les cheveux, **caractérisé en ce qu'**une première composition telle que définie selon l'une quelconque des revendications 1 à 8 est appliquée aux fibres de kératine, suivie par une deuxième composition comprenant au moins un sel métallique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2814943
- FR 2814945
- FR 2814946
- FR 2814947
- FR 2939654
- FR 2939644
- FR 2939645
- FR 2939646
- CN 102824283
- WO 201402670 A


### Non-patent literature cited in the description


- *CHEMICAL ABSTRACTS,* 6485-39-8